# EUROPEAN PATENT APPLICATION

(11) **EP 4 707 796 A1**
(43) Date of publication of application: **11.03.2026**
(21) Application number: 24859154.7
(22) Date of filing: 03.07.2024
(51) Int. Cl.: G01N 33/207, G01N 33/2025

(54) **METHOD FOR MANUFACTURING TEST MATERIAL, METHOD FOR MEASURING DIFFUSIBLE HYDROGEN CONTENT, METHOD FOR EVALUATING DELAYED FRACTURE CHARACTERISTIC, METHOD FOR SELECTING METAL MATERIAL, AND METHOD FOR MANUFACTURING MEMBER**

(30) Priority: 30.08.2023 JP 2023140082
(71) Applicant: JFE Steel Corporation, Tokyo, 100-0011 (JP)
(72) Inventor: MIYAJIMA Ayaka, Tokyo 100-0011 (JP); OKANO Hiroshi, Tokyo 100-0011 (JP); MORIMOTO Minako, Tokyo 100-0011 (JP); OTSUKA Shinji, Tokyo 100-0011 (JP); KAWABE Nao, Tokyo 100-0011 (JP); KIM Jingeum, Tokyo 100-0011 (JP); MATSUDA Hiroshi, Tokyo 100-0011 (JP)
(74) Representative: Grünecker Patent- und Rechtsanwälte PartG mbB
(86) International application number: PCT/JP2024/024016
(87) International publication number: WO 2025/047109

(57) **Abstract**

There is provided a method for producing a test material which can measure with high accuracy the amount of diffusible hydrogen in a metal material having a weld.

The method for producing a test material for measuring the amount of diffusible hydrogen in a metal material having a weld, includes: a blowhole position determination step of determining the position of a blowhole in a test material, which is a metal material having a weld; and a processing step of either processing the test material so that the blowhole, whose position has been determined in the blowhole position determination step, does not have a closed structure, or removing the blowhole.

## Description

### Technical Field

The present disclosure relates to a method for producing a test material, a method for measuring the amount of diffusible hydrogen, a method for evaluating delayed fracture properties, a method for selecting a metal material, and a method for producing a member.

### Background Art

In recent years, from the viewpoint of preventing global warming, there is a demand for improving energy efficiency, for example gasoline fuel efficiency in the case of automobiles, by reducing the weight of moving bodies such as automobiles, ships, and railway cars. A steel material, which is one of constituent materials for moving bodies, is required to have an increased strength in order to ensure the same level of safety even when its thickness is reduced in order to reduce its weight.

On the other hand, a steel material for automobiles is plastically worked into a desired shape by press forming, and such formed steel parts are assembled into a car body by welding, for example, spot welding. Therefore, a steel material for automobiles is not only required to have press formability, but is also required to be capable of being appropriately welded in various steel sheet assemblies.

However, there is a problem that as the strength of steel materials increases, a phenomenon called delayed fracture is more likely to occur. Delayed fracture refers to a phenomenon in which, when a metal material is subjected to a static load over a period of time, a brittle fracture occurs suddenly without any significant apparent plastic deformation, and herein particularly refers to a hydrogen embrittlement fracture caused by entry of hydrogen into a metal material.

Known factors affecting such delayed fracture include the susceptibility of the metal material to hydrogen embrittlement, an applied load (e.g., residual stress), and the amount of hydrogen that has penetrated into the metal material. For example, in the case of a steel material, an increase in the strength of the steel material significantly increases the susceptibility of the steel material to hydrogen embrittlement, which may result in a fracture (cracking) of the steel material even when the amount of hydrogen that has penetrated into the steel material is very small. In particular, a steel material having a tensile strength of 1180 MPa or more has a remarkably increased susceptibility to hydrogen embrittlement (Non-Patent Literature 1).

Therefore, a steel material is required to be free from delayed fracture, i.e., being excellent in delayed fracture resistance, despite the increase in the strength required for the steel material. In particular, a weld of a steel material has a higher strength than the base steel material, and therefore is more susceptible to hydrogen embrittlement. Further, it has been reported that delayed fracture occurs also in metal materials other than steel.

A demand therefore exists for a method to evaluate delayed fracture properties of a weld of a metal material. For example, Patent Literature 1 discloses in the working examples a method for evaluating the hydrogen embrittlement properties of a spot weld of a steel sheet. The method involves holding 2-mm or 4-mm thick sheets as spacers between opposite ends of steel sheets, spot-welding the steel sheets at a position intermediate between the spacers to produce a test specimen, immersing the specimen in 0.5 mol/L sulfuric acid, allowing hydrogen to penetrate into the specimen with an electric current, and evaluating the presence or absence of cracks after two hours.

It is believed that delayed fracture is affected by the penetration of hydrogen (diffusible hydrogen) into a steel material. It is, therefore, conceivable that the delayed fracture properties of a weld can be evaluated by measuring the maximum amount of diffusible hydrogen (limiting diffusible hydrogen amount) that does not cause delayed fracture in the weld.

For example, Patent Literature 2 discloses a diffusible hydrogen amount measuring method which can measure with high accuracy the amount of diffusible hydrogen, which may cause delayed fracture, from a welded joint which is an actual structure.

### Citation List

### Patent Literature

PTL 1: Japanese Unexamined Patent Application Publication No. 2007-231373
PTL 2: Japanese Unexamined Patent Application Publication No. 2008-261821

### Non-Patent Literature

NPL 1: Shinsaku Matsuyama: Delayed Fracture, The Nikkan Kogyo Shimbun, Ltd., Tokyo, (1989)

### Summary of Invention

### Technical Problem

When the delayed fracture properties of a weld of a metal material are evaluated using the method disclosed in Patent Literature 1, it is necessary to conduct a test for a predetermined time and determine whether cracking has occurred in the weld. This method, which determines the occurrence of cracking in the weld after the predetermined time has elapsed, has the problem that a test specimen in which cracking has occurred before elapse of the predetermined time and a test specimen in which cracking has occurred upon elapse of the predetermined time are evaluated as the same level. Thus, the method cannot evaluate with high accuracy the occurrence of cracking in a discriminative manner for such test specimens.

The results of an experiment, which was conducted by introducing hydrogen into welds of welded joints under the same conditions using the method for measuring the amount of diffusible hydrogen in a welded joint, disclosed in Patent Literature 2, and measuring the amount of diffusible hydrogen (amount of hydrogen introduced) in the welds, indicated that measured values for the amount of hydrogen can vary greatly among welds.

The present disclosure has been made in view of the above situation. It is therefore an object of the present disclosure to provide a method for producing a test material which can measure with high accuracy the amount of diffusible hydrogen in a metal material having a weld.

It is also an object of the present disclosure to provide a method for measuring the amount of diffusible hydrogen using a test material produced by the test material production method, a method for evaluating delayed fracture properties, a method for selecting a metal material, and a method for producing a member.

### Solution to Problem

The present inventors, through their intensive studies, found that the above objects can be achieved by the following features, leading to completion of the present invention.
[1] A method for producing a test material for measuring the amount of diffusible hydrogen in a metal material having a weld, including:
   a blowhole position determination step of determining the position of a blowhole in a test material, which is a metal material having a weld; and
   a processing step of either processing the test material so that the blowhole, whose position has been determined in the blowhole position determination step, does not have a closed structure, or removing the blowhole.
[2] The method for producing a test material according to [1], wherein the volume ratio of a weld metal in the test material is 50% or more.
[3] A method for measuring the amount of diffusible hydrogen in a metal material having a weld, including:
   a hydrogen introduction step of introducing hydrogen into a test material produced by the method for producing a test material according to [1] or [2]; and
   a hydrogen amount measurement step of measuring the amount of hydrogen in the test material into which hydrogen has been introduced in the hydrogen introduction step.
[4] A method for evaluating delayed fracture properties of a metal material having a weld, including an evaluation step of evaluating the delayed fracture properties based on the amount of diffusible hydrogen, obtained by the method for measuring the amount of diffusible hydrogen according to [3].
[5] A method for selecting a metal material, including a selection step of selecting a metal material based on the delayed fracture properties obtained by the method for evaluating delayed fracture properties according to [4].
[6] A method for producing a member, including a welding step of at least welding a metal material, selected by the method for selecting a metal material according to [5], to produce a member.

### Advantageous Effects of Invention

The present disclosure can provide a method for producing a test material which can measure with high accuracy the amount of diffusible hydrogen in a metal material having a weld.

The present disclosure can also provide a method for measuring the amount of diffusible hydrogen using a test material produced by the test material production method, a method for evaluating delayed fracture properties, a method for selecting a metal material, and a method for producing a member.

### Brief Description of Drawings

[FIG. 1] FIG. 1 is a schematic diagram illustrating an exemplary method for taking a test material from a metal material having a weld.
[FIG. 2] FIG. 2 shows schematic diagrams illustrating exemplary processing methods for use in a processing step.

### Description of Embodiments

With reference to the above-described problem of poor reproducibility in terms of the amount of diffusible hydrogen in a metal material having a weld, the present inventors have conducted studies using spot-welded steel materials, and have now found that the above-described methods have the following problems.

A blowhole, which is an internal defect, may be present in a spot weld. When a spot weld having a blowhole is exposed to an environment (hydrogen-penetrating environment) in which hydrogen can penetrate into the spot weld, a large amount of hydrogen may accumulate in the blowhole. It is presumed that the hydrogen in the blowhole is gaseous. The hydrogen that has accumulated in the blowhole is stable hydrogen, i.e., non-diffusible hydrogen. According to Non-Patent Literature 1, such non-diffusible hydrogen does not significantly affect delayed fracture properties. However, it is possible that hydrogen that has accumulated in a blowhole may be forcibly desorbed and detected as diffusible hydrogen in thermal desorption spectroscopy of hydrogen, resulting in an apparent increase in the amount of hydrogen (diffusible hydrogen) that has penetrated into a weld. In addition, the amount of hydrogen accumulated in a blowhole varies depending on the size of the blowhole. Thus, the amount of hydrogen as measured for a spot weld varies depending on the volume and number of blowholes. It is difficult for current welding technology to control the size, shape, and number of blowholes.

The present inventors have investigated the relationship between a blowhole(s) present in a weld and the amount of hydrogen in the weld, and found that by eliminating a blowhole(s) having a closed structure, it becomes possible to evaluate with high accuracy the amount of hydrogen (diffusible hydrogen) which has penetrated into a weld from the environment and which affects delayed fracture.

The present disclosure has been made based on the above findings. The following description illustrates a method for producing a test material for measuring the amount of diffusible hydrogen in a metal material having a weld, a method for measuring the amount of diffusible hydrogen, a method for evaluating delayed fracture, a method for selecting a metal material, and a method for producing a member according to embodiments of the present disclosure. It should be noted that the present disclosure is not limited to the following embodiments. It should also be noted that components or elements in the following embodiments include those which are easily replaceable by a person skilled in the art, or those which are substantially the same.

### <Method for Producing a Test Material>

A method for producing a test material for measuring the amount of diffusible hydrogen in a metal material having a weld, according to an embodiment of the present disclosure, includes: a blowhole position determination step of determining the position of a blowhole in a test material, which is a metal material having a weld; and a processing step of either processing the test material so that the blowhole, whose position has been determined in the blowhole position determination step, does not have a closed structure, or removing the blowhole.

### (Metal Material)

The metal material will be described first. The metal material is not particularly limited as long as it is a metal material which is weldable and in which a hydrogen-induced delayed fracture phenomenon may occur. As described above, in the case of a steel material, the susceptibility to hydrogen embrittlement is remarkably high when the tensile strength of the steel is 1180 MPa or more. Therefore, a high-strength steel sheet, particularly a steel sheet having a tensile strength of 1180 MPa or more, is preferred as the metal material. The metal material may be a coated steel sheet including the high-strength steel sheet as a base.

The metal material having a weld may be any welded metal material. Here, the weld consists of a weld metal and a heat-affected zone.

The weld may be formed by any of fusion welding (arc welding, laser welding, electron beam welding, plasma arc welding, etc.) and pressure welding (spot welding, projection welding, seam welding, etc.). When spot welding, for example, is used as a welding method, the weld metal constitutes a nugget portion.

The test material is preferably produced such that the volume ratio of the weld metal is 50% or more. Therefore, a test material production method according to an embodiment of the present disclosure may include a preparation step of preparing a test material containing the weld metal at a desired volume ratio. Hydrogen (diffusible hydrogen) which affects delayed fracture is absorbed in a greater amount into the weld metal than into the base metal and the heat-affected zone. Therefore, in order to appropriately evaluate the delayed fracture properties of a metal material having a weld, the volume ratio of the weld metal in the test material is preferably made 50% or more. The volume ratio of the weld metal is more preferably 70% or more, even more preferably 80% or more, and may be 100%.

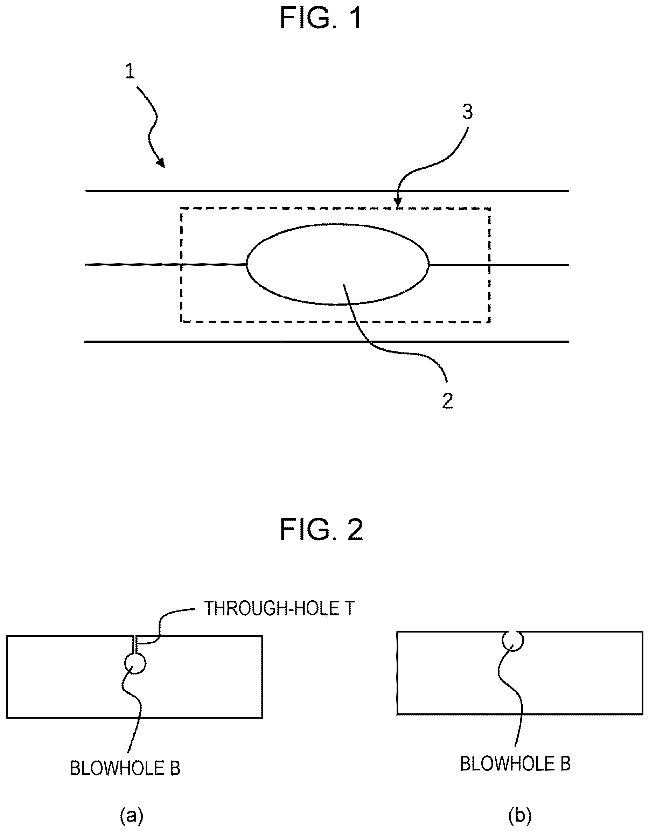

The volume ratio of the weld metal in the test material may be adjusted, for example, when the test material is taken from a metal material after welding (metal material having a weld). FIG. 1 is a schematic diagram illustrating an exemplary method for taking a test material from a metal material having a weld. As shown in FIG. 1, in the preparation step, the test material 3 may be taken from the metal material 1 having a weld such that the test material 3 contains a weld metal (nugget portion) 2. As described above, the test material 3 is preferably taken such that the volume ratio of the weld metal 2 is 50% or more. The test material 3 can be taken by a cutting method using, for example, a micro-cutter or wire cutting. In addition, in the preparation step, the volume ratio of the weld metal contained in the test material may be adjusted by adjusting, for example, a welding current upon welding of the metal material.

In order to properly evaluate superiority or inferiority in delayed fracture properties among metal materials having a weld, it is preferred to equalize the volume ratio of the weld metal among the test materials. Thus, when superiority or inferiority in delayed fracture properties is to be evaluated among test materials, the variation in the volume ratio of the weld metal among the test materials is preferably within the range of ±10%, more preferably within the range of ±5%.

### (Blowhole Position Determination Step)

In the blowhole position determination step, the position of a blowhole present in the weld of the test material is determined. The determination of the position of a blowhole is performed by a non-destructive inspection because it is necessary to introduce hydrogen into the test material and measure the amount of introduced hydrogen after the determination of the position of the blowhole in the weld. The non-destructive inspection is, for example, X-ray CT inspection or ultrasonic inspection. The use of an image, obtained in such an inspection, can also evaluate the volume ratio of the weld metal in the test material.

### (Processing Step)

In the processing step, the test material is either processed so that the blowhole, whose position has been determined in the blowhole position determination step, does not have a closed structure, or processed to remove the blowhole. Exemplary methods for processing the test material so that the blowhole does not have a closed structure include a method which, as shown in FIG. 2(a), involves forming a through-hole T extending from the surface of the test material to the blowhole B, and a method which, as shown in FIG. 2(b), involves grinding or polishing the surface of the test material until the blowhole B becomes connected to the external environment. The test material can thus be processed in such a manner as to allow the blowhole to connect with the external environment, i.e., to make the blowhole not have a closed structure. There is no particular limitation on the method which involves forming the through-hole. For example, a micro-drill or the like may be used to form the through-hole extending from the surface of the test material to the blowhole. There is no particular limitation on the method which involves grinding or polishing the surface of the test material; for example, the surface of the test material may be ground or polished mechanically or chemically. When the blowhole is located in the vicinity of the surface of the test material, the blowhole may be removed by grinding or polishing the surface of the test material. When the test material has an as-cut surface, it is possible that mechanical defects, such as work-hardened areas caused by an increase in dislocations or lattice defects, may be present in the surface, and that hydrogen may be trapped in the mechanical defects. Therefore, it is preferred to chemically polish the test material to remove the as-cut surface.

The test material for measuring the amount of diffusible hydrogen in a metal material having a weld is produced by the procedure described above. The test material produced by the test material production method according to an embodiment of the present disclosure is suitable for use as a test material for measuring the amount of diffusible hydrogen, particularly for use as a test material for evaluating delayed fracture properties.

### <Method for Measuring the Amount of Diffusible Hydrogen>

A method for measuring the amount of diffusible hydrogen in a metal material having a weld, according to an embodiment of the present disclosure, includes: a hydrogen introduction step of introducing hydrogen into a test material produced by the above-described test material production method; and a hydrogen amount measurement step of measuring the amount of hydrogen in the test material into which hydrogen has been introduced in the hydrogen introduction step.

### (Hydrogen Introduction Step)

In the hydrogen introduction step, hydrogen is introduced into the test material produced in the above-described manner. A method for introducing hydrogen may be appropriately selected depending on the purpose. Specific examples of hydrogen introduction methods include a cathode charge method which involves electrolyzing an electrolyte solution by passing therethrough an electric current between the test material as a cathode and platinum or the like as an anode, and introducing hydrogen, generated during the electrolysis, into the test material, and a hydrochloric acid immersion method which involves immersing the test material in a hydrochloric acid solution or the like. When it is desired to evaluate the amount of hydrogen which will be introduced into the metal material in an environment (actual environment) where the material is to be actually used, the test material can be placed in the actual environment. After hydrogen is introduced into the test material in the hydrogen introduction step, the test material is preferably immersed and stored in liquid nitrogen. Immersion of the test material in liquid nitrogen can prevent desorption of hydrogen that has been introduced into the test material.

### (Hydrogen Amount Measurement Step)

In the hydrogen amount measurement step, the amount of hydrogen (diffusible hydrogen) in the test material, into which hydrogen has been introduced in the hydrogen introduction step, is measured. There is no particular limitation on a method for measuring the amount of hydrogen as long as it can measure the amount of hydrogen. For example, thermal desorption spectroscopy can be used. Thermal desorption spectroscopy can analyze a minute amount of hydrogen, and therefore is preferred as an analytical method for measuring the amount of diffusible hydrogen to evaluate the delayed fracture properties of the metal material. In the case where the test material, into which hydrogen has been introduced in the hydrogen introduction step, is stored in liquid nitrogen, the measurement of the amount of hydrogen is preferably performed promptly after removing the test material from the liquid nitrogen.

### <Method for Evaluating Delayed Fracture Properties>

A method for evaluating delayed fracture properties according to an embodiment of the present disclosure includes an evaluation step of evaluating delayed fracture properties based on the amount of hydrogen (diffusible hydrogen), obtained by the above-described method for measuring the amount of diffusible hydrogen. When, for example, superiority or inferiority in the delayed fracture properties of a weld is determined among different test materials of different metal materials, the following evaluation test may be conducted on each test material in the evaluation step. In the hydrogen introduction step of the above-described diffusible hydrogen amount measurement method, the amount of diffusible hydrogen is measured for each test material in the same hydrogen introduction environment (hydrogen introduction conditions), and the measured values for the test materials are compared for evaluation of the delayed fracture properties. It is generally believed that the larger the amount of diffusible hydrogen in a metal material, the more delayed fracture is likely to occur in the metal material. For more accurate evaluation, it is preferred to measure the amount of diffusible hydrogen under hydrogen introduction conditions which cause cracking in a weld of a test material to determine the delayed fracture properties of the test material.

### <Method for Selecting a Metal Material>

A method for selecting a metal material according to an embodiment of the present disclosure includes a selection step of selecting a metal material based on the delayed fracture properties obtained by the above-described method for evaluating delayed fracture properties. By performing the above-described evaluation step on metal materials having a weld, it is possible to acquire a relationship between welding conditions and delayed fracture properties, e.g. the amount of diffusible hydrogen which has been introduced in the same hydrogen introduction environment in the hydrogen introduction step. It is also possible to acquire a relationship between them using various types of metal materials. These relationships can be used to classify metal materials. In the selection step, by evaluating the amount of diffusible hydrogen e.g. in an environment where a metal material is used, it is possible to select, from the classification of metal materials, a metal material suitable for use in the environment. The selected metal material can then be shipped.

### <Method for Producing a Member>

A method for producing a member, according to an embodiment of the present disclosure, includes a welding step of at least welding a metal material, selected by the above-described method for selecting a metal material, to produce a member. The member may be produced by welding the metal material after it is worked, or by working the metal material after it is welded. A welding method for use in the welding step is not particularly limited, and any known welding method can be used. The working is not particularly limited; various metal working processes, such as forming, may be used. The member is preferably an automotive member.

### EXAMPLES

The following examples illustrate the present disclosure in greater detail. It should be noted that the present disclosure is not limited to the examples.

First, metal materials having a weld were produced by the following procedure. Steel sheets A to C, each having a representative chemical composition as shown in Table 1 and having a thickness of 1.6 mm and a tensile strength of 1200 to 1500 MPa, were prepared.

**[Table 1]**

| No. | Metal material | Chemical composition (mass %) | | | | | Tensile strength MPa |
|---|---|---|---|---|---|---|---|
| | | C | Si | Mn | S | P | |
| 1 | Steel sheet A | 0.198 | 0.24 | 1.23 | 0.0005 | 0.008 | 1470 |
| 2 | Steel sheet B | 0.145 | 0.2 | 4.98 | 0.0008 | 0.0034 | 1210 |
| 3 | Steel sheet C | 0.111 | 0.52 | 2.5 | 0.0006 | 0.009 | 1180 |

First, for each of the steel sheets A, B, and C, three test specimens, each of which had been cut to 120 mm × 30 mm, were spot-welded at a position around the center of the test specimens. The spot welding was performed at room temperature using DR-type electrodes, made of chromium copper and having a top diameter of 6 mm and a top curvature radius of 40 mm, as lower and upper electrodes while keeping the electrodes water-cooled. An electric current was applied to the specimens so that the diameter of a nugget formed would be 3√t (t: sheet thickness), thereby producing a metal material (test sample) having a weld.

A test material was taken from each of the thus-obtained test samples using a micro-cutter. The size of the test material was adjusted to meet a target volume ratio of the weld metal. The test material was then chemically polished by immersing it in a mixed solution of hydrogen peroxide and oxalic acid.

### (Blowhole Position Determination Step)

The position of a blowhole(s) in each test material was determined three-dimensionally using X-ray CT. Further, using an image obtained by X-ray CT, the volume ratio of the weld metal in the test material was calculated. The number of the thus-determined blowholes and the calculated volume ratio of the weld metal in the test material are shown in Tables 2 and 3.

### (Processing Step)

Next, the blowhole(s), whose position has been determined in the blowhole position determination step, was processed so that it did not have a closed structure. Specifically, a through-hole(s) was formed from the surface of the test material using a micro-drill. However, such processing was not performed for No. 1 and No. 2 in Table 2, and such processing was performed on only some of the determined blowholes for No. 3 in Table 2. No processing was performed for No. 6 in Table 2 because no blowhole was present in the weld of the test material (Reference Example). Whether a blowhole had a closed structure after such processing was determined by using X-ray CT on the processed test material. Tables 2 and 3 show the number of blowholes having a closed structure present in each test material. A test material, for which the number of blowholes having a closed structure is shown as 0 in Tables 2 and 3, was evaluated as acceptable based on a determination that processing was performed such that no blowhole had a closed structure.

### (Hydrogen Introduction Step)

Hydrogen was introduced into each of the test materials, which had been produced in the above-described manner, under the hydrogen introduction conditions described in Tables 2 and 3. Immediately after the introduction of hydrogen, each test material was immersed and freeze-stored in liquid nitrogen to prevent desorption of the introduced hydrogen.

### (Hydrogen Amount Measurement Step)

Each test material was quickly removed from liquid nitrogen immediately before measuring the amount of hydrogen, and the amount of hydrogen (diffusible hydrogen) was measured by thermal desorption spectroscopy using a low-temperature thermal hydrogen analyzer. The thermal desorption spectroscopy was performed in the temperature range from -50°C to 800°C at a heating rate of 200°C/h. The amount of diffusible hydrogen was determined as a mass fraction (ppm by mass) by integrating the amount of hydrogen measured in the temperature range from -50°C to 200°C, and dividing the integrated amount of hydrogen by the mass of the test material. The amount of hydrogen (diffusible hydrogen), obtained for each test material, is shown in Tables 2 and 3.

**[Table 2]**

| No. | Metal material of test material | Number of blowholes | Number of blowholes having a closed structure | Evaluation of blowholes | Volume ratio of weld metal (%) | Hydrogen introduction conditions | | | Amount of hydrogen (mass ppm) | Remarks |
|---|---|---|---|---|---|---|---|---|---|---|
| | | | | | | Hydrogen introduction method | Solution | Potential (mV vs. SSE) | | |
| 1 | Steel sheet A | 3 | 3 | Unacceptable | 89 | | Aqueous solution of 3 mass % NaCl + 0.1 mass % NH₄SCN | | 4.0 | Comp. Ex. |
| 2 | Steel sheet A | 2 | 2 | Unacceptable | 83 | | | | 3.8 | Comp. Ex. |
| 3 | Steel sheet A | 3 | 1 | Unacceptable | 90 | Cathodic electrolysis | | -1000 | 4.5 | Comp. Ex. |
| 4 | Steel sheet A | 2 | 0 | Acceptable | 81 | | | | 3.2 | Inventive Ex. |
| 5 | Steel sheet A | 1 | 0 | Acceptable | 88 | | | | 2.3 | Inventive Ex. |
| 6 | Steel sheet A | 0 | 0 | - | 84 | | | | 2.7 | Ref. Ex. |

**[Table 3]**

| No. | Metal material of test material | Number of blowholes | Number of blowholes having a closed structure | Evaluation of blowholes | Volume ratio of weld metal (%) | Hydrogen introduction conditions | | | Amount of hydrogen (mass ppm) | Remarks |
|---|---|---|---|---|---|---|---|---|---|---|
| | | | | | | Hydrogen introduction method | Solution | Potential (mV vs. SSE) | | |
| 1 | Steel sheet A | 1 | 0 | Acceptable | 87 | | | | 3.2 | Inventive Ex. |
| 2 | Steel sheet A | 1 | 0 | Acceptable | 72 | | | | 2.9 | Inventive Ex. |
| 3 | Steel sheet A | 2 | 0 | Acceptable | 69 | | | | 2.6 | Inventive Ex. |
| 4 | Steel sheet A | 1 | 0 | Acceptable | 52 | | | | 2.4 | Inventive Ex. |
| 5 | Steel sheet A | 1 | 0 | Acceptable | 45 | | | | 2.2 | Inventive Ex. |
| 6 | Steel sheet B | 1 | 0 | Acceptable | 87 | | Aqueous solution of 3 mass % NaCl + 0.1 mass % NH₄SCN | | 2.5 | Inventive Ex. |
| 7 | Steel sheet B | 1 | 0 | Acceptable | 71 | | | | 2.2 | Inventive Ex. |
| 8 | Steel sheet B | 2 | 0 | Acceptable | 66 | Cathodic electrolysis | | -1200 | 2.2 | Inventive Ex. |
| 9 | Steel sheet B | 1 | 0 | Acceptable | 53 | | | | 2.0 | Inventive Ex. |
| 10 | Steel sheet B | 1 | 0 | Acceptable | 42 | | | | 2.1 | Inventive Ex. |
| 11 | Steel sheet C | 1 | 0 | Acceptable | 84 | | | | 2.0 | Inventive Ex. |
| 12 | Steel sheet C | 1 | 0 | Acceptable | 77 | | | | 2.0 | Inventive Ex. |
| 13 | Steel sheet C | 2 | 0 | Acceptable | 61 | | | | 2.1 | Inventive Ex. |
| 14 | Steel sheet C | 1 | 0 | Acceptable | 50 | | | | 1.9 | Inventive Ex. |
| 15 | Steel sheet C | 1 | 0 | Acceptable | 41 | | | | 1.9 | Inventive Ex. |

As seen in Table 2, when blowholes having a closed structure were present in the test materials (No. 1 to No. 3), the amount of diffusible hydrogen was 3.8 to 4.5 ppm by mass. On the other hand, when a blowhole(s) having a closed structure was eliminated by processing (No. 4, No. 5), the amount of diffusible hydrogen was 2.3 to 3.2 ppm by mass. The amount of diffusible hydrogen in the test material (No. 6) having no blowhole was 2.7 ppm by mass. The results indicate that the amount of diffusible hydrogen is significantly reduced by eliminating a blowhole(s) having a closed structure, and that the amount of diffusible hydrogen is overestimated due to the presence of a blowhole(s) having a closed structure. The results also indicate that the amount of diffusible hydrogen can be evaluated with high accuracy by using a test material from which a blowhole(s) having a closed structure has been eliminated.

As seen in Table 3, in the test materials from which a blowhole(s) having a closed structure had been eliminated, when the volume ratios of the weld metal in the test materials were approximately the same, the measured amounts of diffusible hydrogen were ranked as follows: steel sheet A > steel sheet B > steel sheet C. It was also found that the difference in the amount of diffusible hydrogen between the steel grades was more remarkable when the volume ratios of the weld metal in the test materials were 50% or more.

On the other hand, the delayed fracture properties of a weld were evaluated for the steel sheets A, B, and C using the conventional method (Patent Literature 1). As a result, the delayed fracture properties were ranked as follows: (superior) steel sheet C > steel sheet B > steel sheet A (inferior).

The experimental results thus indicate that the larger the amount of diffusible hydrogen, measured by the method of the present disclosure, in a steel sheet, the more delayed fracture is likely to occur in the steel sheet.

It will be appreciated from the above that the method of the present disclosure can measure the amount of diffusible hydrogen in a metal material having a weld with high accuracy and high reproducibility, and that the method of the present disclosure can be used as an index for evaluating delayed fracture properties of the metal material, and can quantitatively evaluate the delayed fracture properties with high accuracy.

### Reference Signs List

- 1: metal material having a weld
- 2: weld metal
- 3: test material
- B: blowhole
- T: through-hole

## Claims

1. A method for producing a test material for measuring the amount of diffusible hydrogen in a metal material having a weld, comprising:
a blowhole position determination step of determining the position of a blowhole in a test material, which is a metal material having a weld; and
a processing step of either processing the test material so that the blowhole, whose position has been determined in the blowhole position determination step, does not have a closed structure, or removing the blowhole.

2. The method for producing a test material according to claim 1, wherein the volume ratio of a weld metal in the test material is 50% or more.

3. A method for measuring the amount of diffusible hydrogen in a metal material having a weld, comprising:
a hydrogen introduction step of introducing hydrogen into a test material produced by the method for producing a test material according to claim 1 or 2; and
a hydrogen amount measurement step of measuring the amount of hydrogen in the test material into which hydrogen has been introduced in the hydrogen introduction step.

4. A method for evaluating delayed fracture properties of a metal material having a weld, comprising an evaluation step of evaluating the delayed fracture properties based on the amount of diffusible hydrogen, obtained by the method for measuring the amount of diffusible hydrogen according to claim 3.

5. A method for selecting a metal material, comprising a selection step of selecting a metal material based on the delayed fracture properties obtained by the method for evaluating delayed fracture properties according to claim 4.

6. A method for producing a member, comprising a welding step of at least welding a metal material, selected by the method for selecting a metal material according to claim 5, to produce a member.
